# NEW EUROPEAN PATENT SPECIFICATION

(11) **EP 1 411 921 B2**
(45) Date of publication and mention of the opposition decision: **18.08.2010**
(45) Mention of the grant of the patent: 19.12.2007
(21) Application number: 02754918.7
(22) Date of filing: 19.07.2002
(51) Int. Cl.: A61K 31/35, A61K 9/20, A61K 9/50, A61K 9/28, A61K 9/48, A61P 31/10

(54) **PHARMACEUTICAL COMPOSITIONS CONTAINING TERBINAFINE AND USE THEREOF**
ARZNEIZUSAMMENSETZUNGEN ENTHALTEND TERBINAFIN UND DEREN VERWENDUNG
COMPOSITIONS PHARMACEUTIQUES CONTENANT DE LA TERBINAFINE ET LEUR UTILISATION

(30) Priority: 20.07.2001 GB 0117760; 04.12.2001 GB 0128993; 27.05.2002 GB 0212209
(43) Date of publication of application: 28.04.2004
(73) Proprietor: Novartis AG, 4056 Basel (CH); Novartis Pharma GmbH, 1230 Wien (AT)
(72) Inventor: ALLES, Rainer, 88348 Bad Saulgau (DE); BECKER, Dieter, 79102 Freiburg (DE); BONNY, Jean-Daniel, CH-4414 Füllinsdorf (CH); HIRSCH, Stefan, 79539 Lörrach (DE); KALB, Oskar, 79539 Lörrach (DE); KÖLLE, Ernst, Ulrich, 79219 Staufen (DE); MAYER, Friedrich, Karl, CH-4104 Oberwil (CH); STÜTZ, Anton, A-1238 Wien (AT); WILLIAMS, Anthony, CH-4102 Binningen (CH)
(74) Representative: de Weerd, Petrus G.W.
(86) International application number: PCT/EP2002/008095
(87) International publication number: WO 2003/022267

(56) References cited:
- WO-A-01/52895
- WO-A-01/95890
- WO-A-95/16465
- WO-A1-00//16747
- WO-A1-00//44353
- WO-A1-01//37808
- WO-A1-01//62229
- WO-A1-99//26608
- WO-A1-03//022267
- DE-A- 10 017 996
- US- - 4 713 248
- US-A- 5 660 839
- DATABASE WPI Section Ch, Week 200129 Derwent Publications Ltd., London, GB; Class B05, AN 2001-274112 XP002221728 & CN 1 279 944 A (JILU PHARM FACTORY), 17 January 2001 (2001-01-17)
- GB 0117760.9 (PRIO)
- GB 0128993.3 (PRIO)
- GB 0212209.1 (PRIO)
- RUDOLF VOIGT: 'Pharmazeutische Technologie, 9. Auflage', 2000, DEUTSCHER APOTHEKER VERLAG, STUTTGART pages 173, 141 - 142
- P. H. LIST ET AL.: 'Arzneiformenlehre, 4. Auflage', 1985, WVG page 83
- 'Remington's Pharmaceutical Siences, 18th Edition', 1990, MACK PUBLISHING COMPANY, EASTON, PENNSYLVANIA pages 1642,43 - 1436,37

## Description

The invention relates to pharmaceutical compositions of terbinafine, in particular solid dosage forms for oral administration, and their use.
Terbihafine is known from e.g. EP-A-24587. It belongs to the class of allylamine anti-mycotics. It is commercially available under the trademark Lamisil^{R}. Terbinafine is effective upon both topical and oral administration, in a wide range of fungal infections. Terbinafine is particularly useful against dermatophytes, contagious fungi that invade dead tissues of the skin or its appendages such as stratum corneum, nail, and hair.
Terbinafine may be in free base form or in e.g. pharmaceutically acceptable salt form, e.g. the hydrochloride, lactate, ascorbate or malate, e.g. L.(+)-hydrogen malate form.
It preferably is in the hydrochloride acid addition salt form. An acid addition salt form may be prepared from the free base form in conventional manner and vice-versa.

Nail fungi make their home in the nail bed, shielded by the hard outer nail. Thus once the infection is established under the nail, the nail itself provides the fungus with a protective environment that allows it to grow. The effects of these fungi on the nails may be unsightly, seriously complicate foot-care, have a deleterious impact on patients' overall quality of life and well-being and impair the patients' ability to work. If left untreated, the fungi can deform toenails permanently and lead to pain on walking. Additionally the fungi can lead to fissures in the skin, encouraging bacterial infection. Serious complications as a result of these infections may occur in people suffering from diabetes such as diabetic foot syndrome, including primary disease-related complications, e.g. gangrene that, ultimately, can be life-threatening or require amputations. Other high-risk patient sub-groups include patients infected with human immunodeficiency virus (HIV), patients with acquired immunodeficiency syndrome (AIDS), and patients with other types of immunosuppression, e.g. transplant recipients and patients on long-term corticosteroid therapy.

There is an increased prevalence of onychomycosis in the elderly (up to 30 % by age sixty). Microsporum, Trichophyton such as Trichophyton rubrum or Trichophyton mentagrophytes, and Epidermophyton such as Epidermophyton floccosum are those microorganisms commonly involved. These infections are conveniently discussed according to the sites of the body involved. Diagnosis is confirmed by demonstrating the pathogenic fungus in scrapings of the lesions, either by microscopic examination or by culture. Across medical disciplines, onychomycosis is well recognized as being arduous both to diagnose and to manage, particularly in the aged.

Terbinafine is particularly useful to treat toenail and fingernail onychomycosis due to dermatophytes (e.g. tinea unguium). Indeed terbinafine has opened up treatment for tinea ungnium caused by Trichophyton. For example The Merck Manual [1987] states that treatment of toe-nails should be discouraged with the previously used standard griseofulvin, because 1 to 2 years treatment is required, recurrence is usual and complete cure unlikely.

For the treatment of onychomycosis and other uses, terbinafine is normally administered as an immediate release tablet form containing 125 mg or 250 mg terbinafine (base equivalent) once daily. Such a tablet sold under the trademark Lamisil^{R} releases terbinafine to the extent of at least 80 % over a 30-minute period as measured by standard in vitro dissolution studies, e.g. at pH 3 using the paddle method. This is an example of an immediate release form. Terbinafine treatment over 12 weeks is required (hereinafter referred to as the "original treatment period"). The progress of its clinical effectiveness may be seen with growth of the healthy nail, pushing out and replacing the diseased unsightly nail-containing debris and dead fungus. About 10 months is needed for a totally new toe-nail to form.

Although terbinafine is generally regarded as safe like any prescription drug, adverse events associated with its use have been reported. As described in the Physicians' Desk Reference, there have been a number of adverse events recorded, e.g. headaches, gastrointestinal symptoms (including diarrhea, dyspepsia, abdominal pain, nausea and flatulence), liver test abnormalities, e.g. enzyme abnormalities, dermatological symptoms such as pruritis, urticaria and rashes, and taste disturbances, e.g. loss of taste. These adverse events are in general mild and transient. Further adverse events include symptomatic idiosyncratic hepatobiliary dysfunction (e.g. cholestatic hepatitis), severe skin reactions such as Stevens-Johnson syndrome, neutropenia and thrombocytopenia. Yet further adverse events may include visual disturbances such as changes in the ocular lens and retina, as well as allergic reactions including anaphylaxis, fatigue, vomiting, arthralgia, myalgia and hair loss. Terbinafine is a potent inhibitor of CYP2D6 and may cause clinically significant interactions when co-administered with substrates of this isoform such as nortriptyline, desipramine, perphenazine, metoprolol, encainide and propafenone. Hereinafter any and all these events are referred to as "Adverse Events".

Various pharmacokinetic and biopharmaceutical properties of terbinafine are known. Thus terbinafine is well absorbed. Peak drug plasma concentrations (Cₘₐₓ) of about 1 µg/ml appear within 2 hours after administration of a single 250 mg terbinafine dose. The area under the curve over 24 hours (hereinafter AUC) is about 4.56 µg.hour/ml. A moderate increase in AUC is apparent when terbinafine is administered with a meal. In patients with renal impairment (e.g. creatinine clearance up to 50 ml /min) or hepatic cirrhosis the clearance of terbinafine is reduced by approximately 50 %. In the steady state, e.g. when the troughs and peaks are constant after dosing extending over several days, in comparison to the single dose, the peak terbinafine blood concentration (Cₘₐₓ) is 25 % higher and the AUC increases by a factor of 2.5. This is consistent with an effective half-life for terbinafine of about 36 hours.

Pharmacokinetic and absorption properties have been disclosed in e.g. J. Faergemann et al., Acta Derm. Venereol. (Stockh.) 77 [1997] 74-76 and earlier articles. Little has been disclosed on steady-state pharmacokinetics and pharmacokinetics on cessation of steady-state treatment. Although some low aborption was found to occur in the lower gastrointestinal tract, the main site of absorption of terbinafine is not precisely known and as indicated above there is no clinically proven correlation of effect with pharmacokinetic profile.

Further, despite the very major contribution to antimycotic therapy which terbinafine has brought, the reported occurrence of undesirable Adverse Events has been an impediment to its wider oral use or application. The particular difficulties encountered in relation to oral dosing with terbinafine have inevitably led to restrictions in the use of terbinafine therapy for the treatment of relatively less severe or endangering disease conditions, e.g. tinea pedis.

While numerous pharmaceutical compositions for topical and oral administration have been proposed, there still exists a need for commercially acceptable terbinafine formulations for oral administration with good patient convenience and acceptance, especially for children and the elderly. One particular difficulty in the formulation of terbinafine in oral pharmaceutical compositions is its unpleasant, e.g. bitter taste, and/or low physical integrity in free base form. Further, some patients may suffer from taste disturbance or taste loss.

It has now been found that, surprisingly, terbinafine has a beneficial pharmacodynamic profile even in situations of high dosage load. It may therefore be administered without untoward effect on e.g. the liver in higher daily dosage used intermittently and for a shorter duration of time than previously contemplated for the treatment of fungal infections such as onychomycosis or fungal sinusitis, yielding the unexpected result of equal or improved therapeutic outcomes from less total drug exposure, thus resulting in an overall dose of less drug than with previously known, e.g. continuous treatments, e.g. of about 30 % less. Thus the present invention enables reduction of terbinafine treatment times and overall dosing over the full treatment period required to achieve effective therapy, thereby reducing the exposure time to terbinafine and improving the global safety profile.

In addition it permits closer standardization as well as optimization of on-going daily dosage requirements for individual subjects receiving terbinafine therapy as well as for groups of patients undergoing equivalent therapy. By closer standardization of individual patient therapeutic regimens, dosaging parameters for particular patient groups, as well as monitoring requirements, may be reduced, thus substantially reducing the cost of therapy. Further, the antifungal activity of terbinafine being not just fungistatic but fungicidal, it may be used intermittently and administered for a short duration of time while nevertheless being curative, thus largely avoiding the need for prophylactic repeat treatment once mycological cure has been obtained and achieving increased efficacy without corresponding side effects.

The beneficial pharmacodynamic profile of terbinafine appears e.g. from tolerability studies upon high dosage over a short time duration. This is shown in e.g. standard tolerability or pharmacokinetic studies wherein terbinafine in immediate release form, such as a tablet, is administered at dosages higher than usual, namely tolerability studies in beagle dogs effected perorally (p.o.). Pharmacokinetic parameters (toxicokinetics), e.g. tₘₐₓ, Cₘₐₓ, Cₘₐₓ/dose and AUC are measured. The following parameters are also monitored: alanine aminotransferase, albumin, alkaline phosphatase, aspartate aminotransferase, calcium, chloride, total cholesterol, creatine kinase, creatinine, glucose, inorganic phosphorus, magnesium, potassium, sodium, total bilirubin, total protein, triglycerides and urea, as well as gamma glutamyltransferase (GGT). It was found that after a single peroral administration to male dogs of one standard tablet of terbinafine hydrochloride (125 mg base equivalent) at a mean dose of 12.0 ± 0.3 mg/kg terbinafine (base equivalent), the values determined for tₘₐₓ, Cₘₐₓ and Cₘₐₓ/dose were, respectively: 1 h; 199 ± 85 ng/ml; and 16.6 ± 7.2 (ng/ml)/(mg/kg).

Further, it could now be surprisingly determined in extensive computer modeling studies that e.g. in the treatment of onychomycosis, an intermittent dosing of e.g. 350 mg/day terbinafine (base equivalent) administered in 3 cycles, of 14 days on and 14 days off, would result in concentrations in the nail falling between the concentrations achieved with a continuous daily therapy over 12 weeks of, respectively, 125 mg/day, which is known to be less efficacious, and 250 mg/day, which is known to be highly efficacious, in onychomycosis treatment (see Figure). Therefore, it can be concluded that intermittent treatment in the above regimen, or variants thereof, would be expected to produce efficacy in patients.

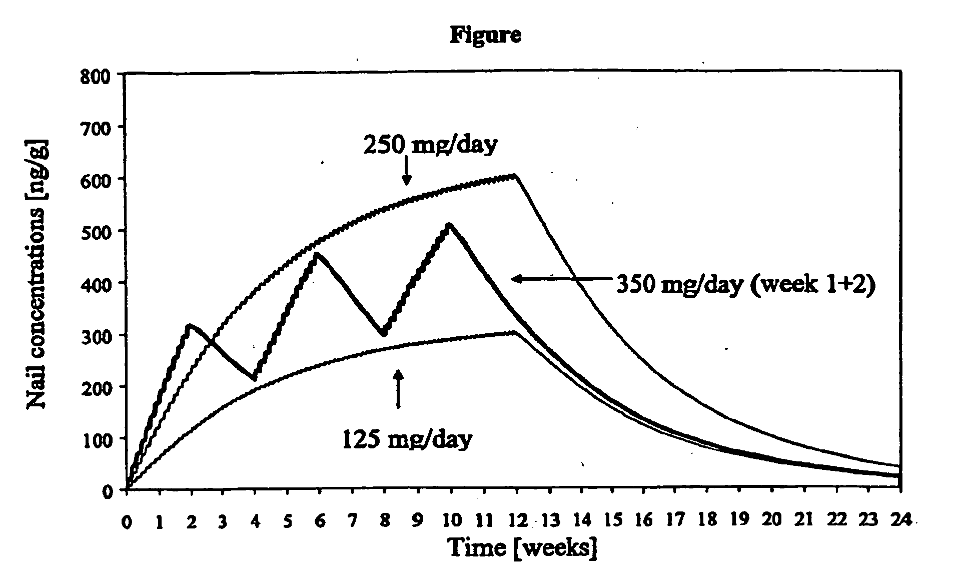

The modeling is effected based on the following principles:
a) Terbinafine plasma concentrations following multiple oral administration is simulated on the basis of known population pharmacokinetic parameters upon continuous therapy [J. Nedelman et al., J.Clin.Pharmacol. 36 (1996) 452-456; J. Nedelman et al., Biopharm.Drug Dispos. 18 (1997) 127-138; and J. Nedelman et al., Eur.J.Drug Metab.Pharmacokinet. 22 (1997) 179-184]. The model incorporates a central, a rapidly equilibrating (shallow) and a slowly equilibrating (deep) peripheral compartment. Drug input into the central compartment is described as a zero order absorption process. Elimination is, as is usual, assumed to occur from the central compartment; and
b) a linear relationship is then established between observed nail concentrations
   [J. Faergemann et al., Acta Derm.Venereol. 73 (1993) 305-309] and the model-predicted drug amount in the deep peripheral compartment. Hence the drug amount in the deep compartment is a suitable predictor for terbinafine nail concentrations.

The invention thus provides a novel terbinafine solid dosage form for oral administration which is suitable for minimizing effects associated with e.g. a high dosage load and wherein the terbinafine particles have a size ranging from about 0,5 mm to about 4 mm in diameter e.g. as easily dispersed particles providing e.g. a reproducible and mainly food-independent transit through the gastrointestinal tract and a high surface area for reproducible dissolution of the drug substance. Such solid dosage form is hereinafter briefly named "the composition(s) of the invention".

The composition of the invention is in the form of mini tablets or pellets.

The constituent particles of the multiparticulate system have a size ranging from about 0.5 mm to about 4 mm in diameter. They are not granules (typically of a particle size of up to about 0.5 mm) but pellets or minitablets. Pellets or minitablets may be filled into capsules, e.g. hard gelatin capsules, or into sachets. Typically, one administration comprises a plurality of pellets or minitablets to achieve the desired overall dose of terbinafine per day.

The particles are minitablets or pellets, i.e. they are presented formulated in a form that allows easy administration of a high load of active substance. The term "minitablets" denotes small tablets with an overall weight in their uncoated form of from about 3 to about 10 mg, e.g. from about 4 to about 7 mg, e.g. about 6 mg. The minitablets may have any shape convenient to the skilled person for tablets, e.g. spherical, e.g. with a diameter of from about 0.5 to about 4 mm, e.g. 1 to 4 mm or 2 to 4 mm; or cylindrical, e.g. having a convex upper face and convex lower face and e.g. with a cylindrical diameter and height which are, independently of each other, of from about 0.5 to about 4 mm, e.g. 1 to 3 mm; or they may be biconvex round minitablets, e.g. whose height and diameter are approximately equal and are from about 0.5 to about 4 mm, e.g. 1.5 to 4 mm, preferably 1.8 to 2.3 mm.

The minitablets are coated with one or more layers of coating.

The coating(s) include(s) a polyacrylate, as taste-masking material, preferably an Eudragit^{R} such as Eudragit^{R}-E or Eudragit^{R}-RD100 or -RS/RL (see Handbook of Pharmaceutical Excipients, loc.cit. hereafter, p. 362), especially Eudragit^{R}-E. In a further variant they are coated with another coating, e.g. with HPMC or polyethyleneglycols (PEG) to minimize further any interaction between minitablet and e.g. capsule. In a further variant the coating is devoid of plasticizer such as dibutyl sebacate, or the plasticizer is a fatty acid such as stearic acid, e.g. stearic acid NF (National Formulary, USP). In a further variant they are unencapsulated. In a further variant in the encapsulating material gelatin is replaced with alternative hard capsule materials, e.g. HPMC or starch.

Similar considerations apply mutatis mutandis for pellets as set out hereabove for minitablets; pellets preferably have a diameter of from about 0.5 to about 2 mm.

The compositions of the invention are formulated in a manner allowing optimal delivery, they are coated. Accordingly, the invention also provides a composition of the present invention in coated form e.g. dragées, or coated tablets, pellets or minitablets, e.g. in capsules. It further provides a composition of the present invention which has taste-masking properties and/or prevents taste disturbance or taste loss and associated adverse effects such as impaired appetite and weight loss.

Suitable coating materials for the compositions of the invention include:
i) pharmaceutically acceptable cellulose derivatives such as ethyl cellulose (EC), hydroxypropyl cellulose (HPC), hydroxypropylmethyl cellulose (HPMC), hydroxypropylmethyl cellulose phthalate (HPMCP) or cellulose acetate phthalate (CAP);
ii) polyacrylates, especially polymethacrylates, preferably:
   a) a copolymer formed from monomers selected from methacrylic acid, methacrylic acid esters, acrylic acid and acrylic acid esters;
   b) a copolymer formed from monomers selected from butyl methacrylate, (2-dimethylaminoethyl)methacrylate and methyl methacrylate; or
   c) a copolymer formed from monomers selected from ethyl acrylate, methyl methacrylate and trimethylammonioethyl methacrylate chloride;
   e.g. those available from Röhm GmbH under the trademark Eudragit^{R};
iii) polyvinyl acetate phthalate (PVAP);
iv) polyvinyl alcohols;
v) polyvinylpyrrolidones (PVP);
vi) sugar such as saccharose or glucose, or sugar alcohols such as xylit or sorbit;
vii) shellac; and
viii) mixtures thereof.

Preferred cellulose derivatives i) are e.g. modified celluloses, e.g. hydroxypropyl cellulose, hydroxyethyl cellulose and hydroxypropylmethyl cellulose, e.g. hydroxypropyl cellulose having a hydroxypropyl content of about 5 to 16 % by weight and of viscosity for 2 % w/w aqueous solutions of from about 2.0 to about 20 cps (= mPa.s), preferably from about 2.0 to about 6.0, e.g. 3.0 cps, e.g. hydroxypropyl methylcellulose (HPMC)
(e.g. USP type 2910, 3 cps), available as e.g. Pharmacoat^{R} 603.
Especially preferred polyacrylic polymers ii) are:
1) the 1:1 copolymers formed from monomers selected from methacrylic acid and methacrylic acid lower alkyl esters, such as the 1:1 copolymers formed from methacrylic acid and methyl methacrylate available under the trademark Eudragit^{R} L, e.g. Eudragit^{R} L100, and the 1:1 copolymer of methacrylic acid and acrylic acid ethyl ester available under the trademark Eudragit^{R} L100-55;
2) the 1:2:1 copolymer formed from butyl methacrylate, (2-dimethylaminoethyl)-methacrylate and methyl methacrylate available under the trademark Eudragit^{R} E; and
3) the 1:2:0.2 copolymer formed from ethyl acrylate, methyl methacrylate and trimethylammonioethyl methacrylate chloride available under the trademark Eudragit^{R} RL; or the corresponding 1:2:0.1 copolymer available under the trademark Eudragit^{R} RS; or the 1:2:0.2 copolymer formed from ethyl acrylate, methyl methacrylate and trimethylammonioethyl methacrylate chloride which is in combination with carboxymethyl cellulose and available under the trademark Eudragit^{R} RD.

The polyacrylates of group 3) above normally contain cationic ester groups. Examples of such cationic groups include dialkylaminoalkyl groups, e.g. dimethylaminoalkyl groups. Especially preferred cationic groups include quaternary ammonium groups, preferably a tri(alkyl)aminoalkyl group. Examples of such groups are trimethylaminoethyl ester groups. The polyacrylate may contain some carboxylic acid groups in free form or salt anions, e.g. chloride anions in order to balance the cationic groups. The ratio of cationic groups to neutral groups is preferably from 1:10 to 1:50, e.g. from 1:10 to 1:30.

The polyacrylates of group ii) above have a mean molecular weight of about 50'000 to about 500'000, e.g. about 150'000.

'Preferably, the coating materials comprise HPMC, Eudragits or sugar. It has been found that polyacrylates ii), especially Eudragit^{k} E, are particularly suitable for coating solid dosage forms comprising terbinafine in the form of the free base as well as in form of its salts, e.g. terbinafine hydrochloride, e.g. since a coating with Eudragit^{R} E does not easily dissolve at the neutral pH of the mouth, but only at pH values below 5, and thereby prevents the dissolution of the bitter tasting terbinafine until transfer to the stomach.

Coating materials as hereinabove defined may be used in admixture with further excipients conventional in coating formulations, for example talcum, magnesium stearate or silicon dioxide, for example synthetic amorphous silicic acid of the Syloid^{R} type (Grace), for example Syloid^{R} 244 FP, or colloidal silicon dioxide, e.g. Aerosil^{R}, e.g. Aerosil^{R} 200, or wetting agents, for example sodium dodecyl sulfate or the aforementioned polyethyleneglycols or polysorbates.

The coating materials may comprise additional excipients, for example plasticisers such as: triethyl citrate, e.g. Citroflex^{R} (e.g. from Morflex); triacetin; various phthalates, e.g. diethyl or dibutyl phthalate; diethyl or dibutyl sebacate; fatty acids or mixtures thereof, e.g. lauric, myristic, palmitic or stearic acid; alcohols, e.g. lauryl or stearyl alcohol; mixed mono- or diglycerides of the Myvacet^{R} type (Eastman), for example Myvacet^{R} 9-40; the polyethyleneglycols mentioned hereinbefore, for example having a molecular weight of approximately from 6000 to 8000; and also ethylene oxide/propylene oxide block copolymers of the poloxamer type, e.g. Pluronic^{R} (BASF) or Synperonic^{R} (ICI) type, such as Pluronic^{R} F68 (poloxamer 188) having a melting point of about 52°C and a molecular weight of about 6800 to 8975, or Synperonic^{R} PE L44 (poloxamer 124); pulverulent mould release agents, for example magnesium trisilicate; starch; or synthetic amorphous silicic acid of the Syloid^{R} type, for example Syloid^{R} 244 FP.

In one embodiment, the solid dosage forms may be coated by one, or preferably by two or more coatings which are applied one after the other. In one aspect, the solid dosage forms may be coated by a first (e.g. protective) coating applied directly upon the solid dosage form, e.g. comprising HPMC, and a second (e.g. taste-masking) coating applied upon the first coating, e.g. comprising Eudragit^{R}, preferably Eudragit^{R} E or Eudragit^{R} RD100, or ethyl cellulose.

In another aspect the solid dosage forms may comprise a further coating, e.g. a layer of anti-sticking material applied upon one of the above-mentioned coatings, e.g. comprising a colloidal silicon dioxide product, e.g. Aerosil^{R}, which may avoid adhesion of the solid dosage forms to each other or to the walls of the container material, e.g. a capsule.

Typically, overall coating weights for coating materials i) to v) range from about 0.5 to about 10 mg/cm² based on the surface area of the uncoated formulation, e.g. from about 1 to about 4 mg/cm², e.g. they are about 1.5 mg/cm². In particularly preferred embodiments, for a 350 mg terbinafine (base equivalent) coated tablet the coat weight is from about 3 to about 14 mg, and for a coated minitablet of about 6.5 mg terbinafine (base equivalent), the coat weight is about from about 0.5 or 1 to about 2 mg.

Typically, overall coating weights for coating materials vi) to vii) range from about 10 to about 200 % of core weight, preferably from about 50 to about 100 % of core weight.

Terbinafine base equivalent may be present in an amount of from about 0.1 to about 95 %, e.g. from about 20 to about 90 %, preferably from about 30 to about 80 %, especially from about 50 to about 60 % by weight based on the total weight of the composition.

The solid dosage forms typically may comprise disintegrants, e.g. such pharmaceutical excipients which facilitate the disintegration of a solid dosage form when placed in an aqueous environment, and may comprise e.g. the following:
(i) natural starches, such as maize starch, potato starch, and the like; directly compressible starches, e.g. Sta-rx^{R} 1500; modified starches, e.g. carboxymethyl starches and sodium starch glycolate, available as Primojel^{R}; Explotab^{R}; Explosol^{R}; and starch derivatives such as amylose;
(ii) crosslinked polyvinylpyrrolidones, e.g. crospovidones, e.g. Polyplasdone^{R} XL and Kollidon^{R} CL;
(iii) alginic acid and sodium alginate;
(iv) methacrylic acid/divinylbenzene copolymer salts, e.g. Amberlite^{R} IRP-88; and
(v) cross-linked sodium carboxymethylcellulose, available as e.g. Ac-di-sol^{R}, Primellose^{R}, Phumacel^{R} XI, Explocel^{R} and Nymcel^{R} ZSX.

Preferred disintegrants include those from classes (i) and (ii) above, particularly preferred are Starx^{R}, Primojel^{R} and Polyplasdone^{R}.

The disintegrant may be present in an amount of from about 1 to about 50 %, e.g. from about 5 to about 40 % by weight based on the total weight of the uncoated composition.

In a further aspect the invention provides a composition of the invention wherein the ratio of terbinafine (base equivalent) to disintegrant is from about 1 : 0.01 to about 1 : 20, e.g. from about 1 : 0.05 to about 1 : 5, preferably from about 1:0.05 to about 1:1 by weight.

The compositions of the invention may also comprise further components which are commonly employed in the preparation of dosage forms, e.g. solid dosage forms. These components include, among others: binders; filler and plasticising agents; lubricants, e.g. magnesium stearate; and glidants, e.g. silica, e.g. in particular colloidal silicon dioxide products available under the trademark Aerosil^{R} (see H.P. Fiedler, loc. cit. hereafter, p. 115; Handbook of Pharmaceutical Excipients, loc. cit. hereafter, p. 424).

Suitable binders include the following:
(i) starches, e.g. potato starch, wheat starch or corn starch;
(ii) gums such as gum tragacanth, acacia gum or gelatin;
(iii) microcrystalline cellulose, e.g. products known under the trademarks Avicel^{R}, Filtrak^{R}, Heweten^{R} or Pharmacell^{R};
(iv) modified celluloses, e.g. hydroxypropyl cellulose, hydroxyethyl cellulose and hydroxypropylmethyl cellulose, e.g. hydroxypropyl cellulose having a hydroxypropyl content of about 5 to 16 % by weight and of viscosity for 2 % w/w aqueous solutions of from about 2.0 to about 20 cps (= mPa.s), preferably from about 2.0 to about 6.0, e.g. 3.0 cps, e.g. hydroxypropyl methylcellulose (HPMC) (e.g. USP type 2910, 3 cps), available as e.g. Pharmacoat^{R} 603; and
(v) polyvinylpyrrolidone, available as e.g. Povidone^{R}, Kollidon^{R} or Plasdone^{R}.

A particularly preferred binder is HPMC (Pharmacoat^{R}). The binder may be present in an amount of from about 0.5 to about 50 %, e.g. from about 1 to about 40 %, e.g. from about 1 to about 25 %, e.g. from about 1 to about 15 %, preferably from about 1 to about 8 % by weight based on the total weight of the uncoated composition.

In a further aspect the invention provides a composition of the invention wherein the ratio of terbinafine (base equivalent) to binder is from about 1 : 0.01 to about 1 : 10, e.g. from about 1 : 0.01 to about 1 : 1, preferably from about 1 : 0.01 to about 1 : 0.1, especially about 1 : 0.04 by weight.

Suitable filler and plasticising agents include excipients known for their favourable properties as filler and plasticising agents, and include:
(i) substantially water-insoluble excipients such as microcrystalline cellulose (which may also be regarded as a weak disintegrant), e.g. Avicel^{R}, Pharmacel^{R}, Emcocell^{R}, Vivapurl^{R}, preferably Avicel^{R} (FMC Corp.), e.g. of the types Avicel^{R} PH101, 102, 105, RC 581 or RC 591 (Fiedler, loc. cit. hereafter, p. 216).
(ii) substantially water-soluble excipients such as compression sugars, e.g. lactose, sucrose, amylose, dextrose, mannitol and inositol, preferably lactose; and
(iii) calcium hydrogen orthophosphate dihydrate, e.g. Emcompress^{R}, or anhydrous calcium hydrogen phosphate, e.g. Fujicalin^{R}.

If present, the filler and plasticising agents may be present in an amount of from about 0.1 to about 50 %, e.g. from about 1 to about 40 %, preferably from about 5 to about 30 % by weight based on the total weight of the uncoated composition.

In a further aspect the invention provides a composition of the invention wherein the ratio of terbinafine (base equivalent) to filler or plasticising agent is from about 1 : 0.01 to about 1 : 100, e.g. from about 1 : 0.01 to about 1 : 20, preferably from about 1 : 0.01 to about 1 : 10, especially from about 1 : 0.1 to about 1 : 5, more especially about 1 : 0.2 by weight.

The compositions of the invention may conveniently further comprise a suitable buffering component, e.g. a salt of an acid that is partially dissociated in aqueous solution, and include those buffering components which - upon disintegration of the composition in an aqueous medium (e.g. the oral cavity) - are capable of maintaining a pH at which terbinafine remains substantially insoluble, e.g. a pH in acidic range, e.g. a pH of greater than 4, preferably of from about 5 to about 6, on treatment with excess water, e.g. 5 to 100 ml. Examples of suitable buffers include carbonate, citrate, acetate, phosphate, phthalate, tartrate salts of the alkali and alkaline earth metal cations, such as sodium, potassium, magnesium and calcium. Preferred buffering agents include e.g. calcium carbonate, trisodium citrate and sodium hydrogen carbonate. The buffering agents may be used singly or in any suitable combination for achieving the desired pH and may be of a buffer strength of from about 0.01 to about 1 mole/litre, preferably from about 0.01 to about 0.1 mole/litre.

The compositions of the invention are adapted for release of the active substance terbinafine in the stomach; for example, in 0.04 M citrate buffer pH 3.0 at 37°C, terbinafine is released from the composition and dissolves within 30 minutes to the extent of at least 50 %, e.g. at least 70 %, preferably at least 80 %.

The molar ratio of terbinafine (base equivalent) to buffering component may be from about 1 : 0.02 to about 1 : 10, e.g. from about 1 : 0.2 to about 1 : 10, preferably from about 1 : 0.5 to about 1 : 5, more preferably from about 1 : 0.5 to about 1 : 2.

It will be appreciated that the invention encompasses:
a) in respect of the disintegrant any of components i) to v) individually or in combination with one or more of the other components i) to v);
b) in respect of the binder and filler or plasticizing agent any of those specified above individually or in combination; and
c) in respect of the buffering component any of the buffers specified above individually or in combination.

The compositions may conveniently also include one or more further additives or ingredients in an amount of e.g. from about 0.01 to about 5 % by weight based on the total weight of the uncoated composition, for example: sweetening agents, e.g. sorbitol, saccharin, aspartame, acesulfame or sugars such as glucose, fructose or saccharose; flavouring agents, e.g. chocolate, cocoa, banana, strawberry or vanilla flavour; and so forth. Additives to sugar or shellac coating commonly used in confectioning may be employed where appropriate.

Determination of workable proportions in any particular instance will generally be within the capability of the man skilled in the art. All indicated proportions and relative weight ranges described above are accordingly to be understood as being indicative of preferred or individually inventive teachings only and not as limiting the invention in its broadest aspect.

Compositions of the invention are coated minitablets or pellets wherein the coating comprises a (taste-masking) polyacrylate coating, preferably Eudragit^{R} E or Eudragit RD100^{R}, especially Eudragit^{R} E, whereby the polyacrylate coating and the terbinafine-containing core optionally are separated by a readily-dissolving (protective) coating of, preferably, a cellulose derivative such as HPMC, and optionally further coated with a layer preventing sticking of the minitablets or pellets to each other or to the capsule shell, e.g. comprising colloidal silica such as Aerosil^{R} 200; most especially preferred are the compositions of Examples 4, 5, 6 and 7, preferably Examples 4 and 5, especially Example 5.

In a subgroup the (taste-masking) polyacrylate coating is separated from the core by a readily-dissolving (protective) coating as decribed above.

In yet another aspect the invention provides a process for preparing a coated composition of the invention as defined above, comprising appropriately coating a corresponding uncoated precursor form of a composition of the invention, using conventional methods, e.g. as described in Remington's Pharmaceutical Sciences, 18th Edition, Ed. Alfonso R. Gennaro, Easton, PA : Mack (1990); and in K. Bauer et al., Überzogene Arzneiformen (1988), Wissensch. VG, Stuttgart; the contents of which are incorporated herein. E.g. a coating system may be used in e.g. a conventional non-perforated pan or in a perforated pan by the Accela Cota method, or the submerged sword coating method or fluid bed coating method may be used.

The compositions of the invention thus obtained have an acceptable taste and thus have particularly good patient convenience and patient acceptance due to their increased ease of administration and ingestion. Furthermore, the compositions of the invention, preferably those that are in coated form, prevent taste disturbance or taste loss, probably by preventing terbinafine interference with taste receptors in the oral cavity, in particular on the tongue.

Thus, the compositions of the invention, which are conveniently in solid form, e.g. in the form of a coated tablet or of coated pellets or minitablets, or dragées (i.e. tablets coated with a coating containing sugar and/or sugar alcohols), preferably in the form of a coated tablet or coated minitablets or pellets, may be administered as such or, if desired, e.g. with coated pellets or minitablets, dispersed (but preferably not substantially dissolved) prior to administration in a small amount of a liquid or semi-liquid, e.g. water, milk, yoghurt or juice, e.g. in a spoon.

In addition the compositions of the invention show surprisingly high physical and chemical stability, e.g. for up to two or more years. The physical and chemical stability may be tested in conventional manner, e.g. the compositions may be tested as such by measurement of dissolution, disintegration time, and/or by hardness test, e.g. after storage at room temperature, i.e. at 25°C, and/or after storage at 40°C. The taste of the compositions may be tested in standard clinical studies.

The particles of the multiparticulate system of the invention, i.e. minitablets or pellets may be packaged in conventional manner, e.g. in a bottle, or worked-up into optionally coloured capsules. Such capsules may be in e.g. two parts, and each part may conveniently be of a different colour.

The compositions of the invention are useful for the known indications of terbinafine, e.g. for the following conditions: onychomycosis caused by dermatophyte fungi, fungal sinusitis, tinea capitis, fungal infections of the skin, for the treatment of tinea corporis, tinea cruris, tinea pedis, and yeast infections of the skin caused by the genus Candida, e.g. Candida albicans, systemic mycosis, mycosis by azole-resistant strains, e.g. in combination with a 14-α-methyldimethylase inhibitor, or infections with Helicobacter pylori.

The compositions are particularly effective in treating onychomycosis.

It further provides the use of a composition of the invention in the manufacture of a medicament for the treatment of fungal infections of the human body, in particular of onychomycosis.

It further provides the use of a composition of the invention in the manufacture of a medicament for inhibiting or reducing taste disturbance or taste loss and associated adverse effects such as impaired appetite and weight loss after terbinafine intake.

It further provides the use of a composition of the invention in the manufacture of a medicament for use in the method of the invention as defined above.

The utility of the compositions of the invention may be observed in standard bioavailability tests or standard animal models, for example ascertaining dosages giving blood levels of terbinafine equivalent to blood levels giving a therapeutical effect on administration of known terbinafine oral dosage forms, e.g. a tablet. Typical doses are in the range of from about 1 mg/kg to about 10 mg/kg, e.g. from about 1.5 mg/kg to about 5 mg/kg, or e.g. from about 3 to about 4 mg/kg body weight of terbinafine base equivalent per day. The appropriate dosage will, of course, vary depending upon, for example, the host and the nature and severity of the condition being treated. However in general satisfactory results in animals are indicated to be obtained at daily treatments with doses from about I mg/kg to about 10 mg/kg animal body weight. In humans an indicated daily dosage is in the range of from about 10 mg to about 1000 mg per day, conveniently administered, for example, in divided doses up to four times a day or once daily. Preferred dosages for children weighing less than 20 kg may be about 62.5 mg once daily, for children weighing from 20 to 40 kg about 125 mg once daily, for children weighing more than 40 kg about 250 mg once daily, and for adults from about 250 mg to about 500 mg once daily.

Terbinafine may be administered in immediate release form, e.g. as a tablet or capsule, e.g. a tablet comprising 350 mg base equivalent of active substance, or e.g. one or two capsules with minitablets or pellets comprising 350 mg base equivalent of active substance in total, or in sustained release form. Immediate release forms are preferred.

Suitable sustained release forms are described in Pharmazeutische Technologie, Thieme Verlag, Stuttgart/New York, 2nd Edition [1991], Ed. H. Sucker, P. Fuchs, P. Spieser, e.g. on p. 370-390. Further systems are described in e.g. Pharmaceutical Dosage Forms Ed. Herbert A. Lieberman, Leon Lachman, Joseph B. Schwartz, 2nd edition, Vol. 3, Marcel Dekker; and Remington, The Science and Practice of Pharmacy, Ed. Alfonso Gennaro, 19th Edition [1995]. A wide variety of sustained release systems may be used.

Details of excipients useful in compositions for use in the present invention are known, e.g. from the presently commercialized forms of Lamisil^{R}, or as described in H.P. Fiedler, "Lexikon der Hilfsstoffe für Pharmazie, Kosmetik und angrenzende Gebiete", Editio Cantor Verlag Aulendorf, Aulendorf, 4th revised and expanded Edition (1996); or in " Handbook of Pharmaceutical Excipients", 2nd Edition, Ed. A. Wade and P.J. Weller (1994), Joint publication of American Pharmaceutical Association, Washington, USA and The Pharmaceutical Press, London, England; or may be obtained from brochures from the relevant manufacturers, the contents of which are hereby incorporated by reference.

The amount of terbinafine in a composition of the invention will of course vary, e.g. depending on to what extent other components are present In general, however, the terbinafine will be present in an amount within the range of from 10 % to about 80 % by weight based on the total weight of the composition. Compositions will preferably be compounded in unit dosage form, e.g. by filling into capsule shells, e.g. soft or hard gelatin capsule shells or by tabletting or other moulding process. Thus unit dosage terbinafine composition, suitable for administration once or twice daily (e.g. depending on the particular purpose of therapy, the phase of therapy, etc.) will appropriately comprise half or the total daily dose contemplated. Preferably the compositions of the invention are administered once-a-day.

The pharmacokinetic properties of the compositions of the invention may be determined in standard animal and human pharmacological (bioavailability) trials.

For example one standard pharmacological trial may be carried out in healthy male or female non-smoking volunteers aged between 18 to 45 years having within 20 % of the ideal body weight. Blood samples are taken for 1, 2, 4, 8, 16, 32 and 72 hours post-administration in the method of the invention and tested for terbinafine. Terbinafine blood plasma concentrations may be determined in conventional manner, e.g. by HPLC or GLC analytical techniques. Safety is judged according to a standard checklist based on Adverse Event symptoms after 1 week.

A further standard pharmacological trial is e.g. a bioavailability/food-effect study in a randomized, open-label, three-period, crossover study to evaluate the relative bioavailability of a composition of the invention, e.g. the capsules of Example 5 or 8, compared with standard terbinafine immediate release tablets and to assess the effect of food on the pharmacokinetics of the compositions of the invention after a single dose in 24 healthy adult subjects. The study includes the following three treatments:
- treatment A: 250 mg single standard immediate release tablet under fasted conditions;
- treatment B: 350 mg capsule (2 x 175 mg) of Example 5 under fasted conditions; and
- treatment C: 350 mg capsule (2 x 175 mg) of Example 5 under fed conditions.

For each of the three treatment periods, safety assessments are performed and blood samples collected at defined timepoints until 96 h post-dosing to determine i.a. terbinafine tₘₐₓ, Cₘₐₓ and AUC (area under the curve).

Pharmacokinetic drug skin and nail concentration studies may be carried out according to the same principles as set out for the above-mentioned standard pharmacological trials.

A therapeutic clinical trial may be effected based on the principles of the standard pharmacological trials mentioned above. For example, a randomized double-blind positive-controlled and placebo-controlled study may be effected with subjects having onychomycosis of the toe-nail confirmed by microscopy and culture. Treatment is carried out preferably with three 28-days or monthly cycles in the method of the invention, using the 175 mg capsules of Example 5, and with the original treatment over 12 weeks. Clinical trials may be effected in several hundred patients to ascertain the freedom from Adverse Events. However therapeutic efficacy may be shown in trials with 25 patients aged over 12 years. Safety is evaluated by an Adverse Event report of clinical aspects and vital signs. Efficacy is determined by microscopy, culture procedures and visually looking at signs and symptoms. Efficacy is seen in patients with the fungi described above, especially Trichophyton rubrum, Trichophyton mentagrophytes and Epidermophyton floccosum. Patients include those with predisposing factors such as impaired blood circulation, peripheral neuropathy, diabetes mellitus, damage from repeated minor trauma, and limited immune defects as well as AIDS. Patients have (i) distal lateral subungual onychomycosis starting at the hyponychium spreading proximally to the nail bed and matrix, (ii) proximal subungual onychomycosis, wherein the fungus infects the cuticle and eponychium to reach the matrix where it becomes enclosed into the nail plate substance, (iii) total dystrophic onychomycosis, and (iv) superficial white onychomycosis. If desired serum concentrations of terbinafine may be evaluated in conventional manner. Concentrations of terbinafine in the nail may be evaluated by both photo-acoustic spectroscopy and nail clipping followed by analysis, indicating presence of terbinafine in the nail-bed.

Clinical trials may be effected in particular sub-sets of subjects, e.g. those with impaired renal or hepatic function. Changes in the standard clinical chemistry parameters measured for liver dysfunction are lower than expected for the method of the invention. It is also found that any such dysfunctions are transient and functional. This indicates the excellent tolerability of the compositions of the invention.

The compositions for use in the method of the invention are useful for the same indications as for known immediate release terbinafine tablets, e.g. fungal sinusitis and onychomycosis. The utility of compositions of the invention may be observed in standard clinical tests or standard animal models.

The compositions in the method of the invention are particularly and surprisingly well tolerated with regard to the Adverse Events mentioned above, provoking fewer Adverse Events than would be expected in the original treatment with the standard 250 mg immediate release Lamisil^{R} tablet. From the clinical trials it is seen that the compositions of the invention are just as efficacious particularly in aged patients, e.g. of 70 years and above, in patients with renal impairment (e.g. creatinine clearance ≥ 50 ml/min) or hepatic cirrhosis, and yet tend to provoke surprisingly fewer Adverse Events than expected for the dose given. Moreover the variation in AUC between fasted and fed state is less than expected.

The following Examples illustrate the invention. They are not limitative. All temperatures are in degrees Centigrade. The following abbreviations are used:
- HPMC =: hydroxypropylmethylcellulose
- MW =: molecular weight
- PEG =: polyethyleneglycol

### Example 1 to 3: Coated minitablets

Minitablets are prepared in conventional manner by aqueous granulation of a part of the ingredients, mixing with the other ingredients at dry stage, compressing and coating the resultant minitablets with an aqueous dispersion of the coating ingredients. The resultant biconvex round minitablets have a diameter of about 2.0 to 2.1 mm:

| | **Example 1** | | **Example 2** | | **Example 3** | |
|---|---|---|---|---|---|---|
| Components | % of total mass | mg/mini-tablet | % of total mass | mg/mini-tablet | % of total mass | mg/mini -tablet |
| **Core:** | | | | | | |
| Terbinafine hydrochloride* | 63.80 | 4.6875 | 64.17 | 4.6875 | 63.42 | 4.6875 |
| HPMC 603 (USP type 2910, 3 cps) | 2.65 | 0.1950 | 2.67 | 0.1950 | 2.64 | 0.1950 |
| Microcrystalline cellulose | 10.96 | 0.8050 | 11.02 | 0.8050 | 10.89 | 0.8050 |
| Sodium carboxymethyl starch | 10.17 | 0.7475 | 10.23 | 0.7475 | 10.11 | 0.7475 |
| Colloidal silica | 0.44 | 0.0325 | 0.44 | 0.0325 | 0.44 | 0.0325 |
| Magnesium stearate | 0.89 | 0.0653 | 0.89 | 0.0653 | 0.88 | 0.0653 |
| Total weight (of uncoated minitablet) | | 6.5328 | | 6.5328 | | 6.5328 |
| | | | | | | |

| **Coating 1:** | | | | | | |
|---|---|---|---|---|---|---|
| HPMC 603 (USP type 2910,3 cps) | 2.14 | 0.1570 | 2.15 | 0.1570 | 2.26 | 0.1671 |
| PEG (nominal MW 8000) | 0.43 | 0.0314 | 0.43 | 0.0314 | 0.45 | 0.0334 |
| Silicic acid (Syloid 244FP) | 1.71 | 0.1256 | 1.72 | 0.1256 | 1.80 | 0.1331 |
| | | | | | | |

| **Coating 2:** | | | | | | |
|---|---|---|---|---|---|---|
| Eudragit E PO^{R} (powder) | 4.27 | 0.3140 | 4.30 | 0.3140 | 4.52 | 0.3342 |
| Sodium dodecyl sulfate | 0.28 | 0.0206 | 0.28 | 0.0206 | 0.30 | 0.0220 |
| Dibutyl sebacate | 0.56 | 0.0413 | 0.57 | 0.0413 | 0.60 | 0,0440 |
| Magnesium stearate | 1.12 | 0.0825 | 1.13 | 0.0825 | 1.19 | 0.0880 |
| | | | | | | |

| **Coating 3:** | | | | | | |
|---|---|---|---|---|---|---|
| Colloidal silica | 0.57 | 0.0417 | - | - | 0.49 | 0.0363 |
| Total weight (of coatings per minitablet) | | 0.8141 | | 0.7724 | | 0.8581 |
| | | | | | | |
| Total weight (of coated minitablet) | 100 | 7.3469 | 100 | 7.3052 | 100 | 7.3909 |

| | | | | | | |
|---|---|---|---|---|---|---|
| *corresponds to 4.1667 mg terbinafine base | | | | | | |

For use in the present invention for intermittent cycling, e.g. 84 minitablets (350 mg terbinafine base equivalent) are administered once a day for 14 consecutive days of each cycle.

### Example 4: Hard gelatin capsules comprising doubly-coated minitablets with an anti-sticking laver

### a) Minitablets:

Minitablets are prepared in conventional manner by aqueous granulation of a part of the ingredients, mixing with the other ingredients at dry stage, compressing and coating the resultant minitablets with an aqueous dispersion of the coating ingredients. The resultant biconvex round minitablets have a diameter of about 2.0 to 2.1 mm:

| Components | Amount (mg/minitablet) |
|---|---|
| **Inner phase:** | |
| Terbinafine hydrochloride* | 4.6875 |
| HPMC 603 (USP type 2910, 3 cps) | 0.1950 |
| Microcrystalline cellulose | 0.3325 |
| Sodium carboxymethyl starch | 0.5850 |
| Colloidal silica (Aerosil 200^{R}) | 0.0325 |
| **Outer phase:** | |
| Microcrystalline cellulose | 0.4725 |
| Sodium carboxymethyl starch | 0.1625 |
| Magnesium stearate | 0.0653 |
| | |

| **Coating 1** (protecting): | |
|---|---|
| HPMC 603 (USP type 2910, 3 cps) | 0.10026 |
| PEG (nominal MW 8000) | 0.02004 |
| Colloidal silica (Aerosil 200^{R}) | 0.07986 |
| Purified water** | 2.03340 |
| | |

| **Coating 2** (taste-masking): | |
|---|---|
| Eudragit E PO^{R} (powder) | 0.33420 |
| Sodium lauryl sulfate | 0.02200 |
| Dibutyl sebacate | 0.04400 |
| Magnesium stearate | 0.08800 |
| Purified water** | 1.60380 |
| | |

| **Anti-sticking layer:** | |
|---|---|
| Colloidal silica (Aerosil 200^{R}) | 0.03625 |
| | |
| Total weight (of coated minitablet) | 7.25741 |

| | |
|---|---|
| * corresponds to 4.1667 mg terbinafine base **removed during manufacturing process | |

### b) Capsules:

Coated minitablets obtained as described under a) above are filled into optionally coloured hard gelatin capsules in conventional manner.

For use in the present invention for intermittent cycling, e.g. one capsule containing 84 minitablets (1 x 350 mg) or two capsules containing 42 minitablets each (2 x 175 mg) (350 mg terbinafine base equivalent in total) is administered once a day for 14 consecutive days of each cycle.

### Example 5: Hard gelatin capsules comprising minitablets with reduced protecting coating

Minitablets are prepared and formulated into capsules and may be used for intermittent cycling as described in Example 4, but using for coating 1, 0.02662 mg colloidal silica (Aerosil 200^{R}) in place of 0.07986 mg (total weight: 7.20417 mg/minitablet).

### Examples 6 and 7: Hard gelatin capsules comprising minitablets coated for taste-masking but devoid of protecting coating

Minitablets are prepared and formulated into capsules and may be used for intermittent cycling as described in Example 4, except that the minitablets prepared are devoid of protecting coating 1 (total weight 7.05725 mg/minitablet) (Example 9); in a variant, they are devoid of protecting coating 1, and taste-masking coating 2 is devoid of Eudragit E^{R}, sodium lauryl sulfate and dibutyl sebacate and has the following composition:

| **Example 10** | Amount (mg/minitablet) |
|---|---|
| **Coating 2** (taste-masking): | |
| Magnesium stearate | 0.06684 |
| Polysorbate 80^{R} | 0.06684 |
| Eudragit RD100^{R} | 0.33420 |
| Purified water* | 2.20572 |
| | |
| Total weight (of coated minitablet) | 7.03693 |

| | |
|---|---|
| * removed during manufacturing process | |

### Example 8: Hard gelatin capsules comprising minitablets with enhanced protecting coating

Minitablets are prepared and formulated into capsules and may be used for intermittent cycling as described in Example 4, but for coating 1 (protecting) the amounts of HPMC and PEG 8000 are trebled (0.30078 and 0.06012 mg/minitablet, respectively), and 6.10020 mg purified water (removed during manufacturing process) is used in place of 2.03340 mg (total weight 7.49801 mg/minitablet).

### Examples 9 to 11: Hard gelatin capsules comprising minitablets with modified coating 2

Minitablets are prepared and formulated into capsules and may be used for intermittent cycling as described in Example 4, but for coating 1 (protecting) the amount of colloidal silica (Aerosil 200^{R}) is reduced (0.02662 mg/minitablet in place of 0.07986 mg/minitablet) and for coating 2 (taste-masking) the following ingredients are used:

| Component | Amount (mg/minitablet) | | |
|---|---|---|---|
| | **Example 9** | **Example 10** | **Example 11** |
| Coating 2 (taste-masking): | | | |
| Eudragit E PO^{R} (powder) | 0.3342 | 0.3342 | 0.3342 |
| Sodium lauryl sulfate | 0.03342 | none | none |
| Dibutyl sebacate | none | none | 0.01671 |
| Magnesium stearate | 0.11726 | 0.16710 | 0.16710 |
| Stearic acid | 0.04749 | none | none |
| PEG 8000^{R} | none | 0.03342 | 0.03342 |
| Ethanol | none | 2.88749 | 2.87746 |
| Purified water* | 3.03360 | 1.92499 | 1.91831 |
| | | | |
| Total weight (coated minitablet) | 7.24834 | 7.25069 | 7.26740 |

| | | | |
|---|---|---|---|
| * removed during manufacturing process | | | |

### Examples 12 and 13: Hard gelatin capsules comprising minitablets with modified antisticking layer

Minitablets are prepared and formulated into capsules and may be used for intermittent cycling as described in Example 4, but using for coating 1 (protecting) 0.02662 mg colloidal silica (Aerosil 200^{R}) in place of 0.07986 mg, and for the anti-sticking layer, replacing most (Example 12) or all (Example 13) of the colloidal silica with the following ingredients:

| Component | Amount (mg/minitablet) | |
|---|---|---|
| | **Example 15** | **Example 16** |
| Anti-sticking layer: | | |
| HPMC 603^{R} | 0.05013 | none |
| PEG 8000^{R} | 0.01002 | 0.07350 |
| Colloidal silica (Aerosil 200^{R}) | 0.01331 | none |
| Purified water* | 1.01670 | 0.66150 |
| | | |
| Total weight (of coated minitablet) | 7.24138 | 7.24142 |

| | | |
|---|---|---|
| * removed during manufacturing process | | |

### Example 14: Coated pellets

Coated pellets are prepared in conventional manner by aqueous granulation of the pellet components, extrusion of the wet granulate, spheronization, drying and coating with an aqueous dispersion of the coating components. The resultant pellets have a particle size between about 0.8 and 1.0 mm and have the following composition:

| Components | Amount (g/100 g coated pellets) |
|---|---|
| **Core:** | |
| Terbinafine hydrochloride* | 42.591 |
| Fujicalin^{R} | 31.517 |
| Microcrystalline cellulose | 8.518 |
| Sodium carboxymethyl starch | 2.555 |
| Purified water** | 46.850 |
| | |

| **Coating** (taste-masking): | |
|---|---|
| Eudragit E PO^{R} (powder) | 8.518 |
| Sodium lauryl sulfate | 0.608 |
| Dibutyl sebacate | 1.272 |
| Magnesium stearate | 3.429 |
| Purified water** | 41.485 |
| | |

| **Anti-sticking layer:** | |
|---|---|
| Colloidal silica (Aerosil 200^{R}) | 0.990 |
| | |
| Total weight (of coated pellets) | 100.00 |

| | |
|---|---|
| * corresponds to 37.859 mg terbinafine base per 100 g coated pellets **removed during manufacturing process | |

For use in the present invention for intermittent cycling, e.g. 924.5 mg coated pellets (350 mg terbinafine base equivalent) are administered once a day for 14 consecutive days of each cycle.

### Example 15: Hard gelatin capsules comprising coated pellets

Coated pellets obtained as described in Example 14 above are filled into optionally coloured hard gelatin capsules in conventional manner.

For use in the present invention for intermittent cycling, e.g. two capsules containing 462.25 mg coated pellets each (2 x 175 mg terbinafine base equivalent) or three capsules containing 308.16 mg coated pellets each (3 x 116.67 mg terbinafine base equivalent) (350 mg terbinafine base equivalent in total) are administered once a day for 14 consecutive days of each cycle.

## Claims

1. A terbinafine solid dosage form for oral administration, which is coated and multiparticulate comprising pellets or minitablets, which have a size ranging from 0.5 mm to 4 mm in diameter and are in coated form comprising a polyacrylate coating.

2. A dosage form of claim 1 wherein the minitablets or pellets are in capsules.

3. A dosage form of any one of claims 1 or 2 wherein the minitablets or pellets are in sachets.

4. A dosage form of any one of claims 1 to 3 in which terbinafine is released and dissolved within 30 minutes to the extend of at least 50% in 0.04 M citrate buffer pH 3.0 at 37° C.

5. A dosage form of claim 1, optionally in capsules or sachets, wherein the coating comprises a polyacrylate coating, whereby the polyacrylate coating and the terbinafine-containing coating core are separated by a readily-dissolving coating, and optionally further coated with a layer preventing sticking.

6. Use of a dosage form of any one of claims 1 to 5 in the manufacture of a medicament for the treatment of fungal infection of the human body, in particular onychomycosis.

7. Use according to claim 6 wherein onychomycosis is caused by dermatophyte fungal sinusitis, tinea capitis, fungal infections of the skin, tinea corporis, tinea cruris, tinea pedis, yeast infections of the skin caused by the genus Candida, systemic mycosis, mycosis caused by azole-resistant strains or infections with Helicobacter pylori.

8. Use of a dosage form of any one of claims 1 to 5 in the manufacture of a medicament for reducing taste disturbance or taste loss and associated adverse effects after terbinafine intake.

## Patentansprüche

1. Feste Terbinafindosierungsform für eine orale Verabreichung, die beschichtet und multipartikulär ist, umfassend Pellets oder Minitabletten, die eine Größe in einem Durchmesserbereich von 0,5 mm bis 4 mm haben und in beschichteter Form vorliegen, umfassend einen Polyacrylatüberzug.

2. Dosierungsform nach Anspruch 1, worin die Minitabletten oder Pellets in Kapseln vorhanden sind.

3. Dosierungsform nach einem der Ansprüche 1 oder 2, worin die Minitabletten oder Pellets in Sachets vorhanden sind.

4. Dosierungsform nach einem der Ansprüche 1 bis 3, worin das Terbinafin freigesetzt und gelöst wird innerhalb von 30 min bis zum Ausmaß von wenigstens 50 % in 0,04 molarem Citratpuffer vom pH 3,0 bei 37°C.

5. Dosierungsform nach Anspruch 1, optional in Kapseln oder Sachets, worin die Beschichtung eine Polyacrylatbeschichtung umfasst, wobei die Polyacrylatbeschichtung und der Terbinafin enthaltende Beschichtungskern durch eine sich rasch auflösende Beschichtung getrennt und optional mit einer ein Kleben verhindernden weiteren Beschichtungsschicht versehen sind.

6. Verwendung einer Dosierungsform nach einem der Ansprüche 1 bis 5 zur Herstellung eines Arzneimittels für die Behandlung einer Pilzinfektion des menschlichen Körpers, insbesondere von Onychomykose.

7. Verwendung nach Anspruch 6, worin die Onychomykose verursacht wird durch Dermatophyten, fungale Sinusitis, Tinea capitis, Pilzinfektionen der Haut, Tinea corporis, Tinea cruris, Tinea pedis, Hefeinfektionen der Haut, die durch die Gattung Candida verursacht werden, systemische Mykose und Mykose, die durch Azol-resistente Stämme oder Infektionen mit Helicobacter pylori verursacht wird.

8. Verwendung einer Dosierungsform nach einem der Ansprüche 1 bis 5 zur Herstellung eines Arzneimittels für die Erniedrigung einer Geschmacksstörung oder eines Geschmacksverlusts und assoziierte nachteiliger Effekte nach einer Einnahme von Terbinafin.

## Revendications

1. Forme pharmaceutique solide de terbinafine pour administration par voie orale, enrobée et multiparticulaire, comprenant des granulés ou des mini-comprimés dont le diamètre est compris entre 0,5 mm et 4 mm, et qui se présentent sous forme enrobée, notamment avec un enrobage de polyacrylate.

2. Forme pharmaceutique selon la revendication 1, dans laquelle les mini-comprimés ou les granulés sont contenus dans des capsules.

3. Forme pharmaceutique selon la revendication 1 ou 2, dans laquelle les mini-comprimés ou les granulés sont contenus dans des sachets.

4. Forme pharmaceutique selon l'une des revendications 1 à 3, dans laquelle la terbinafine est libérée et dissoute en l'espace de 30 minutes à hauteur d'au moins 50 % dans un tampon citrate 0,04 M, pH 3,0, à 37°C.

5. Forme pharmaceutique selon la revendication 1, éventuellement contenue dans des capsules ou des sachets, dans laquelle l'enrobage comprend un enrobage de polyacrylate, l'enrobage de polyacrylate et le noyau de l'enrobage contenant la terbinafine étant éventuellement séparés par un enrobage à dissolution rapide et éventuellement enrobés d'une couche supplémentaire qui les empêche de coller.

6. Utilisation d'une forme pharmaceutique selon l'une des revendications 1 à 5 dans la fabrication d'un médicament destiné à traiter une infection fongique du corps humain, tout particulièrement l'onychomycose.

7. Utilisation selon la revendication 6, où l'onychomycose est causée par le champignon dermatophyte, la sinusite fongique, la teigne tondante microscopique, les infections fongiques de la peau, l'herpès circiné, l'eczéma marginé, tinea pedis, les lévuroses causées par le genre *Candida,* la mycose généralisée, la mycose causée par les souches résistant aux azolés ou par des infections par *Helicobacter pylori.*

8. Utilisation d'une forme pharmaceutique selon l'une des revendications 1 à 5 dans la fabrication d'un médicament destiné à réduire la perturbation du goût ou la perte de goût ainsi que les effets secondaires associés consécutifs à la prise de terbinafine.
